# EUROPEAN PATENT SPECIFICATION

(11) **EP 1 778 179 B1**
(45) Date of publication and mention of the grant of the patent: **02.04.2014**
(21) Application number: 05774814.7
(22) Date of filing: 18.08.2005
(51) Int. Cl.: A61K 8/67, A61Q 5/00, A61Q 19/00

(54) **Skin care products containing 6-O-PUFA ascorbic esters for protecting skin damages during exposure to UV radiation**
6-O-PUFA Ascorbinsäureester enthaltende Hautpflegeprodukte zum Schutz vor Hautschäden während UV-Strahlenexposition
Produits pour les soins de la peau contenant des esters 6-O-PUFA ascorbiques pour la protection de la peau contre le lesions provoquées par les rayons UV.

(30) Priority: 18.08.2004 JP 2004237942
(43) Date of publication of application: 02.05.2007
(73) Proprietor: Suntory Holdings Limited, Kita-ku, Osaka-shi Osaka 530-8203 (JP)
(72) Inventor: FUKAMI, Harukazu, Kyoto-shi, Kyoto 6018373 (JP); KAWASHIMA, Hiroshi, Takatsuki-shi, Osaka 5691121 (JP); ONO, Yoshiko, Osaka-shi, Osaka 5330022 (JP)
(74) Representative: Vossius & Partner
(86) International application number: PCT/JP2005/015383
(87) International publication number: WO 2006/019186

(56) References cited:
- EP-A- 0 675 120
- WO-A-99/32105
- GB-A- 991 390
- US-A- 5 078 989
- US-A1- 2002 142 019
- DATABASE CAPLUS [Online] CHEMICAL ABSTRACTS SERVICE, COLUMBUS, OHIO, US; MORI,KENJI ET AL.: "Cosmetics containing ascorbyl gamma-linolenate" XP002353344 retrieved from STN Database accession no. 1987:483706 & JP 62 081307 A (KANEBO LTD) 4 October 1985 (1985-10-04)
- DATABASE CAPLUS [Online] CHEMICAL ABSTRACTS SERVICE, COLUMBUS, OHIO, US; KITAOKA, SHOZABURO ET AL.: "preparation of physiologically active unsaturated fatty acid esters of ascorbic acid or erythrobic acid" XP002353345 retrieved from SNT Database accession no. 1991-631125 & JP 03 099073 A (HARIMA CHEMICALS INC.) 12 September 1989 (1989-09-12)
- DATABASE CAPLUS [Online] CHEMICAL ABSTRACTS SERVICE, COLUMBUS, OHIO, US; ROVESTI PAOLO: "Use of cholesteryl arachidonate and ascorbyl arachidonate in cosmetics" XP002353346 retrieved from STN Database accession no. 1969:31628
- ROVESTI P: "IMPIEGHI COSMETOLOGICI DEGLI ARACHIDONATI DI COLESTERILE E DI ASCORBILE//USE OF CHOLESTERYL ARACHIDONATE AND ASCORBYL ARACHIDONATE IN COSMETICS", RIVISTA ITALIANA ESSENZE, PROFUMI, PIANTE OFFICINALI, AROMI,SAPONI, COSMETICI, AEROSOL, RIVISTA ITALIANA ESSENZE. P.A.S.C.A., MILANO, IT, vol. 50, no. 8, 1 January 1968 (1968-01-01), pages 432-434, XP009056856, ISSN: 0035-6948

## Description

### FIELD OF THE INVENTION

This invention relates to 6-O-PUFA ascorbate and to skin care products that contain 6-O-PUFA (polyunsaturated fatty acid) ascorbic esters as an ascorbic acid component for use in a method for protecting skin from being damaged during exposure to UV radiation, which allow for improved permeability of ascorbic acid to the epidermis or dermis of the skin, in particular, improved transfer of ascorbic acid to skin keratocytes.

Vitamin C promotes the synthesis of collagen whose shortage is a primary cause of scurvy, works as *in vivo* antioxidant to scavenge free radicals that are produced in the living body, and take part in the redox reaction of an iron ion as catalyzed by cytochrome c. In addition to these physiological actions, vitamin C is known to have many other actions such as cancer control, immunopotentiation, and arteriosclerosis control from suppression of cholesterologenesis. In the dermal region, vitamin C which has actions such as preventing photoaging on the basis of anti-oxidation and promoted collagen synthesis, preventing ultraviolet damage, and suppressing pigmentation, is added to cosmetics (FRAGRANCE JOURNAL, Vol. 25, March, Special Feature, page 7, 1997). Vitamin C is also added as an antioxidant in foods and cosmetics. However, if one wants to exploit the physiological functions of vitamin C in cosmetics, its water solubility is so high that it finds difficulty in passing through the dermis of the skin to reach the target cell.

Speaking of PUFAs, docosahexaenoic acid (DHA), eicosapentaenoic acid (EPA), as well as α- or γ-linolenic acid, dihomo-γ-linolenic acid (DGLA) and arachidonic acid are known not only as biological components but also as substances having a variety of beneficial functions ("Nou no hataraki to shishitu", ed. by H. Okuyama et al., Gakkai Center Kansai, 1997; "Kinousei shishitsu no kaihatsu", compiled under the supervision of K. Sato et al., 1992; "Kanzobyo to chiryo eiyou", by A. Watanabe et al., Daiichi Shuppan, 1992). Some of those PUFAs are used in medicines and foods, in particular, health foods. Among those PUFAs, DGLA and GLA are known as precursors for prostaglandin of series 1 (PGE1). PGE1 is known to be antagonistic, for example, to prostaglandin of series 2 (PGE2) to provide an anti-inflammatory effect, as well as showing a suppressive effect on delayed allergy ("Shokubutsu shigen no seirikassei busshitsu handbook", ed. by A. Yoshizumi et al., Science Forum, page 536, 1998).

Ascorbic acid derivatives having PUFA attached to 6-position by an ester linkage are known. They are not only anticipated to exhibit the functions of both PUFA and ascorbic acid; some of the functions of such ascorbic acid derivatives are also known, among which are that 6-0-docosahexaenoyl ascorbate has an antiarrythmic action (JP 10-139664 A), calcium antagonism (WO 94/20092), and an anti-allergic action (JP 6-122621 A), and that 6-O-γ-linolenoyl ascorbate has an aldose reductase inhibitory action (USP 6069168) and is effective in a streptozotocininudced diabetic model (Diabetologia, 1996, 39, 1047). It is also known that the ascorbic acid derivatives having PUFA attached to 6-position by an ester linkage are more resistant to oxidation than the unmodified PUFAs (J. Am Oil Chem. Soc., 2001, 78, 823). Linoleic acid was mixed with maltodextrin, gum arabic or water-soluble polysaccharides such as a soybean's and spray-dried to form microcapsules which were shown to have higher resistance to oxidation than those prepared with linoleic acid per se (J. Agr. Food Chem., 2002, 50, 3984; J. Microencapsulation, 2002, 19, 181). Thus, the ascorbic acid derivatives having PUFA attached to 6-position by an ester linkage have been verified or speculated to have better properties in physiological functional aspects and resistance to oxidation.

However, no attempt has been made to produce skin care products in which ascorbic acid derivatives having PUFA attached to 6-position by an ester linkage are used as an ascorbic acid component.

### SUMMARY OF THE INVENTION

An object of the present invention is to provide skin care products that are anticipated to find application in the field of cosmetics and which, compared to conventional skin care products containing vitamin C per se, are significantly improved in vitamin C incorporation to the cells in the epidermis or dermis of the skin, whereby the functions of ascorbic acid are exhibited efficiently.

Another object of the present invention is to provide skin care products that also show functions based on the physiological activity of PUFAs.

The skin care products described herein contain as a vitamin C component 6-O-PUFA ascorbates represented by general formula (I):

RCO-A (I)

where RCO- is an acyl group derived from a polyunsaturated fatty acid, and A is a residue of ascorbic acid that binds by -O- derived from the hydroxyl group in ascorbic acid.

The present invention is summarized in the following items:
1. A 6-O-PUFA ascorbate for use in a method for protecting skin from being damaged during exposure to UV radiation, wherein the permeability of ascorbic acid into cells in the dermis of the skin is improved, wherein the 6-0-PUFA ascorbate is 6-O-dihomo-γ-linolenoyl ascorbate or 6-O-arachidonoyl ascorbate.
2. A skin care product containing a 6-O-PUFA ascorbate for use in a method for protecting skin from being damaged during exposure to UV radiation, wherein the permeability of ascorbic acid into cells in the dermis of the skin is improved, wherein the 6-0-PUFA ascorbate is 6-O-dihomo-γ-linolenoyl ascorbate or 6-O-arachidonoyl ascorbate and wherein the 6-O-PUFA ascorbate is contained in an amount of 0.001-10 wt% in a base for cosmetics for external application.
3. The skin care product according to item 2, which is selected from the group consisting of lotions, creams, toners, facial packs, cleansers, makeup cosmetics, scalp and hair care products, ointments, dispersions, and solutions for external application.
4. The skin care product according to item 2 or 3, wherein the skin care product does not comprise a transdermal absorption enhancer.
5. Use of a 6-O-PUFA ascorbate for the manufacture of a skin care product for protecting skin from being damaged during exposure to UV radiation, wherein the permeability of ascorbic acid into cells in the dermis of the skin is improved, wherein the 6-O-PUFA ascorbate is 6-O-dihomo-γ-linolenoyl ascorbate or 6-O-arachidonoyl ascorbate.
6. The use according to item 5, wherein the skin care product does not comprise a transdermal absorption enhancer.

### BRIEF DESCRIPTION OF THE DRAWINGS

Fig. 1 is a graph showing the effect of 6-O-dihomo-γ-linolenoyl ascorbate on the concentration of intracellular ascorbic acid in human skin keratocytes.
Fig. 2 is a graph showing the protective effect of 6-O-dihomo-γ-linolenoyl ascorbate on the apoptosis of human skin epidermis derived keratocytes (HaCaT) under irradiation with UVA rays.
Fig. 3 is a graph showing the effect of 6-O-dihomo-γ-linolenoyl ascorbate on the incorporation of ascorbic acid to a skin tissue.

### DETAILED DESCRIPTION OF THE INVENTION

Since vitamin C is water-soluble, its permeability into cells is not necessarily good although its transporter is known; hence, the effect of vitamin C has not been fully exhibited even if it is contained as an active ingredient in skin care products. However, the present inventors have found surprisingly that when PUFA ascorbic acid derivatives having vitamin C esterified with PUFA at 6-position are brought into contact with a skin section, their transfer to the epidermis or dermis is markedly improved over the transfer of vitamin C itself. The inventors also found that when the ester derivatives were added to a culture medium of skin keratocytes being cultivated, the concentration of intracellular vitamin C was markedly increased over the case where vitamin C itself was added. Furthermore, the inventors found that when human skin keratocytes into which the ester derivatives had been taken up were irradiated with UVA, the viability of the cells was markedly improved over the case where vitamin C itself was taken up. The present invention has been accomplished on the basis of these findings.

The skin layer consists of an epidermis and the underlying dermis, with the topmost layer of the epidermis is composed of keratocytes. Therefore, the skin care product for use according to the present invention which contains as a vitamin C component an ascorbic acid derivative of general formula (I) which has PUFA attached to 6-position by an ester linkage, wherein the 6-O-PUFA ascorbate is 6-0-dihomo-y-linolenoyl ascorbate or 6-O-arachidonoyl ascorbate has the advantage that when applied to the skin, it allows more vitamin C to be taken up into keratocytes than conventional skin care products containing vitamin C per se, so that vitamin C is transferred to the epidermis or dermis to enrich the vitamin C in those cells, and that it can be used to protect the skin from being damaged during outdoor exposure to UVA.

It has been verified or speculated that the ascorbic acid derivatives within cells which have PUFAs attached,to 6-position by an ester linkage function both as PUFAs and as vitamin C, with the added ability to improve the stability for oxidation of PUFAs. Further, as represented by DHA, EPA and arachidonic acid, PUFAs are known to have not only physiological functions as components of the living body but also a variety of other beneficial functions. Therefore, the skin care products for use according to the present invention are anticipated to exhibit the following advantages:
A) They will produce vitamin C under the action of cellular esterase or lipase. Vitamin C has outstanding physiological functions as a water-soluble vitamin and it also exhibits a skin whitening action and a skin protecting action against UVA as an outstanding antioxidant.
B) PUFAs that form with vitamin C, for example, DGLA and GLA, will in turn generate PGE1 which exhibits an anti-inflammatory action in antagonism with PGE2 or shows a suppressive action against delayed allergy; in particular, the anti-inflammatory action of DGLA is anticipated to be beneficial to rough skin; the concurrently generated vitamin C works as an antioxidant and will enhance the stability of DGLA and GLA.
C) The functions of the ascorbic acid derivatives themselves which have PUFAs attached to 6-position by an ester linkage are exhibited, for example, the antiarrhythmic, calcium antagonistic and anti-allergic actions if 6-0-docosahexaenoyl ascorbate is used as an ascorbic acid derivative, and the aldose reductase inhibitory action if 6-O-γ-linolenoyl ascorbate is used.

Examples of PUFAs (polyunsaturated fatty acids) that may be used in skin care products and food/beverages include fatty acids having 18 or more carbon atoms and two or more unsaturated bonds, linolenic acid, linolenoic acid, γ-linolenic acid, dihomo-γ-linolenic acid, arachidonic acid, eicosapentaenoic acid and docosahexaenoic acid. The specific examples of the ascorbic acid derivatives represented by general formula (I) which have PUFAs attached to 6-position by an ester linkage for use according to the present invention are 6-O-dihomo-γ-linolenoyl ascorbate and 6-O-arachidonoyl ascorbate.

The ascorbic acid derivatives represented by general formula (I) which have PUFAs attached to 6-position by an ester linkage can be produced by a chemical method of synthesis or an enzymatic method of production, both being known in the art. In the chemical method of synthesis, PUFA and ascorbic acid are condensed with an ordinary dehydrating agent such as dicyclohexyl carbodiimide or an acid chloride of PUFA is treated with ascorbic acid (USP 6069168). In the enzymatic method of production, PUFA and ascorbic acid are dissolved in an organic solvent such as acetone, followed by synthesis with lipase (JAOCS, 2001, 78, 823). The ascorbic acid derivatives produced by such methods which have PUFAs attached to 6-position by an ester linkage may optionally be purified by silica gel chromatography.

The PUFA ascorbic acid derivatives represented by general formula (I) may be formulated as the skin care products either independently or as incorporated in bases for various dosage forms of cosmetics known as ordinary compositions for external application. In the latter case, the PUFA ascorbic acid derivatives may be dissolved in suitable organic solvents before being incorporated in the bases, or they may be directly mixed with liquid bases or base ingredients. The PUFA ascorbic esters are contained in amounts of 0.001-10 wt%, more preferably 0.01-10 wt%, of the base.

The dosage form of the skin care products is not limited in any particular way but they may be formulated as, for example, makeup cosmetics such as lotions, creams, toners, facial packs, cleansers and lipsticks, cosmetics such as scalp and hair care products, and medicated cosmetics such as ointments, dispersions, cream and solutions for external application. Bases for compositions for external application depend on the dosage form of such compositions for external application and may include purified water, lower alcohols, polyols, and fats and oils. The skin care products may contain common auxiliary ingredients such as surfactant, pH modifying component, UV absorber, UV scattering agent, thickener, dye, pigment, antiseptic, flavoring agent, etc. The skin care products may optionally contain physiologically active ingredients other vitamin C and/or nutrient ingredients.

The skin care products may further contain transdermal absorption enhancers in order to enhance the takeup of the vitamin C component into the skin. However, the ascorbic acid derivatives which have PUFAs attached to 6-position by an ester linkage can permeate through the skin very efficiently, so skin care products using no transdermal absorption enhancers are a preferred embodiment.

Herein also described are foods and beverages that contain 6-O-PUFA ascorbic esters. In general, 6-0-PUFA ascorbic esters can be incorporated as a vitamin C component in any foods and beverages. While the amount in which they can be incorporated has no particular limitation, 6-0-PUFA ascorbic esters are generally incorporated in amounts of 0.001-10 wt% of the total quantity of the food or beverage in order to ensure that they exhibit the effectiveness of adding vitamin C. The 6-0-PUFA ascorbic esters may be dissolved in suitable organic solvents before they are incorporated in foods or beverages or, alternatively, they may be directly mixed with liquid beverages.

The foods and beverages may be foods for infants, foods for elderly people, nutritional supplementary foods, intravenous feeding fluids, handy foods for carrying around, sports drinks, vitamin supplements, foods and beverages for pet animals, feeds for cattle, mineral water, seasonings, dairy products, furikake (various fish and vegetables flakes to sprinkle on cooked rice), dashi (soup stock made from kelp, or dried bonito flakes, or both), soft drinks, powdered drinks and alcoholic beverages.

The skin care products in which the ascorbic acid derivatives which have PUFAs attached to 6-position by an ester linkage are contained as a vitamin C component can markedly increase the incorporation of vitamin C into cells, thereby enabling vitamin C to exhibit its functions efficiently.

When the skin care products are applied to the human skin, ascorbic acid will be transferred markedly to the epidermis or dermis and accumulated markedly within the cells of these tissues. In addition, the viability of human skin keratocytes which are vulnerable to UVA damage is improved.

The ascorbic acid derivatives in the skin care products which have PUFAs attached to 6-position by an ester linkage are also anticipated to show the functions of PUFAs. For example, DGLA and GLA are known as precursors of prostaglandin of series 1, with PGE1 being antagonistic to prostaglandin of series 2 (PGE2), working to provide an anti-inflammatory effect. PGE1 is also anticipated to be effective against rough skin. The PUFA ascorbic esters, when they are decomposed in vivo, get the vitamin C component to cause a marked improvement in the stability for oxidation of PUFAs which are highly sensitive for oxidation.

Since the 6-0-PUFA ascorbic esters are improved in permeability into dermal cells, the foods and beverages are also anticipated to permit ease with which the 6-O-PUFA ascorbic esters are absorbed by the small intestinal tract. It is also anticipated that the 6-0-PUFA ascorbic esters which have transferred into the living body will be further incorporated to the epithelial tissue. On the other hand, the 6-0-PUFA ascorbic esters are slowly decomposed to ascorbic acid and PUFA with in vivo esterase or lipase. Therefore, compared to vitamin C, the 6-0-PUFA ascorbic esters are anticipated to ensure that the effect of vitamin C will be sustained longer in the living body. Vitamin C enhances the stability of the resulting PUFAs which, as in the aforementioned case of cosmetics, are anticipated to exhibit not only a variety of healthful actions such as a delayed allergy suppressing action and an anti-inflammatory action, but also an anti-arrhythmic action.

### EXAMPLES

The present invention is described below more specifically on the basis of the following examples, which are by no means intended to limit the scope of the invention. In addition, productions of ascorbic acid esters which have various PUFAs attached to 6-position by an ester linkage are set forth below as reference examples. The compound 6-O-docosahexaenoyl ascorbate used in several Examples as well as Examples 1 and 2 are not within the claims.

### Reference Example 1:

### Synthesis of 6-O-arachidonoyl ascorbate

Arachidonic acid (2.0 g, 6.6 mmol) was dissolved in benzene (20 ml) and after addition of oxalyl chloride (5.4 ml, 7.9 mmol), the mixture was stirred for 2.5 hours at room temperature in a nitrogen atmosphere. Subsequent concentrating under reduced pressure gave an oil of arachidonyl chloride. To a solution of N-methylpyrrolidone (15 ml) in a 4N HCl/dioxane mixture (2.4 ml), L-ascorbic acid (1.4 g, 7.9 mmol) was added and the solution was cooled with ice. To the ice-cooled solution, a solution (ca. 2 ml) of the already prepared arachidonyl chloride in methylene chloride was added and the mixture was stirred overnight under cooling with ice. After the end of the reaction, water was added and extraction with ethyl acetate was performed. The ethyl acetate layer was washed with water (twice), dried with anhydrous magnesium sulfate, and concentrated under reduced pressure. The resulting residue was purified by silica gel chromatography (eluant, 1%-20% gradient) and evaporated to dryness under reduced pressure to give a paste of the titled compound (2.7 g, yield: 90%). PMR(δ ppm, CDCl₃); 0.87(3H, t), 1.2-1.4(6H, m), 1.73(2H, q), 2.0-2.2(4H, m), 2.36(2H, t), 2.8-2.9(6H, 4.2-4.3(3H, m), 4.79(1H, s), 5.36(8H, m).

### Reference Example 2:

### Synthesis of 6-O-dihomo-γ-linolenoyl ascorbate

Dihomo-γ-linolenoic acid (2.0 g, 6.6 mmol) was similarly treated to give a paste of the titled compound (2.43 g, yield: 80%). PMR(δ ppm, CDCl₃); 0.89(3H, t), 1.2-1.4(12H, m),1.63(2H, t), 2.0-2.1(4H, m), 2.39(2H, t), 2.7-2.9(4H, m), 4.2-4.3(3H, m), 4.79(1H, s), 5.2-5.4(6H, m)

### Reference Example 3:

### Synthesis of 6-O-docosahexaenoyl ascorbate

Docosahexaenoic acid (2.0 g, 6.1 mmol) was similarly treated to give a paste of the titled compound (2.5 g, yield: 84%). PMR(δ ppm CDCl₃); 0.97(3H, t), 2.07(2H, q), 2.43(4H, q), 2.8-2.9(10H, m), 4.76(1H, s), 5.2-5.4(10H, m), 4.2-4.7(3H, m), 4.80(1H, s), 5.2-5.5(12H, m).

### Example 1:

### The effect of 6-O-dihomo-γ-linolenoyl ascorbate on the concentration of intracellular ascorbic acid in human skin keratocytes

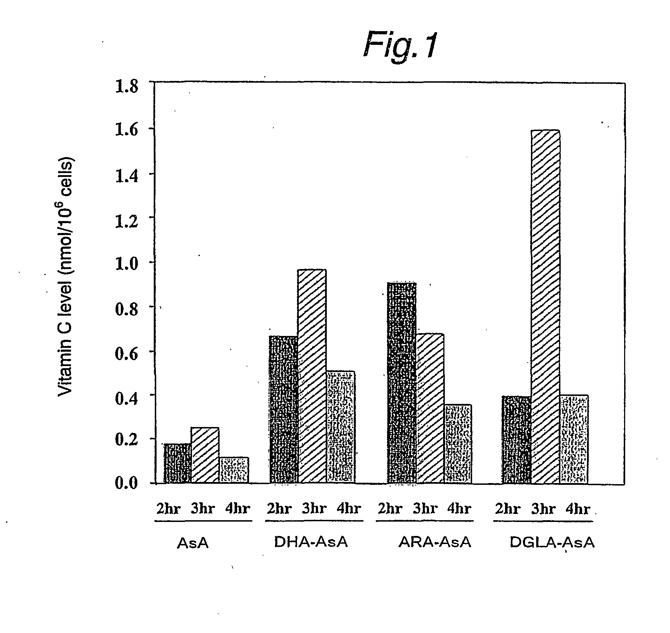

A predetermined number (370,000) of human skin keratocytes HaCaT were inoculated on a 100 mm^{φ} dish. After 16 hours of the culture, 6-O-dihomo-γ-linolenoyl ascorbate dissolved in a 10% FBS containing DMEM supplemented with 40% of a 24-hr serum-free culture solution of HaCaT was added in an amount of 100 µM. Three to 24 hours after the addition, the medium was removed, rinsed with ice-cooled PBS twice, and treated with trypsin to detach the cell sheet comprising single cells. The cells were suspended in PBS containing 50 µM of dithiothreitol (DTT) and centrifugally rinsed three times. The cell suspension was disrupted with a potter-type Teflon homogenizer and subjected to two freeze-thaw cycles in liquid nitrogen. The supernatant was treated with Molcut (pressurized ultrafiltration unit produced by Nihon Millipore Corporation; fractionating molecular weight, 10,000; polyether sulfone membrane) and subjected to high-performance liquid chromatography [AS-8020 System produced by TOSOH CORPORATION; column, Shodex ODSpak (product of SHOWA DENKO K.K.; 4.6 x 150 mm); mobile phase, 0.1M KH₂PO₄-H₃PO₄ (pH 2.35)-0.1 mM EDTA-2Na; flow rate, 1.5 mL/min], and the amount of intracellular ascorbic acid was assayed with a coulometric electrochemical detector (ESACo, Bedford, MA, 200 mV). The results are shown in Fig. 1.

Similarly, 6-O-arachidonoyl ascorbate and 6-0-docosahexaenoyl ascorbate, as well as ascorbic acid (comparison) were evaluated and the results are also shown in Fig. 1.

As Fig. 1 shows, the amount of vitamin C in human skin keratocytes was more increased by the PUFA ascorbic acid derivatives than by unmodified vitamin C.

### Example 2:

### The protective effect of 6-O-dihomo-γ-linolenoyl ascorbate on the apoptosis of human skin epidermis derived keratocytes (HaCaT) under irradiation with UVA rays.

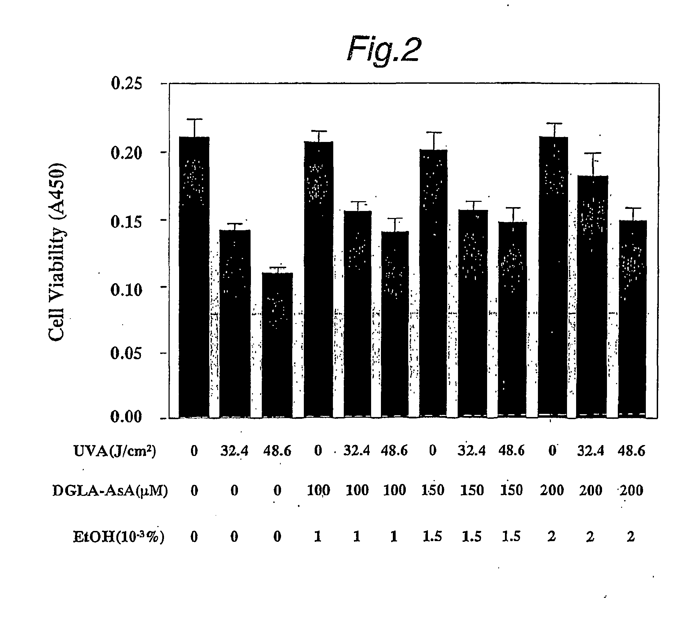

Human skin keratocytes HaCaT (the cell line granted by courtesy of Dr. Fusenig at University of Heidelberg) were inoculated on a 24-well plate at a density of 10,000 cells/well in a 10% fetal bovine serum (FBS) containing Dulbecco modified Eagle medium (DMEM); 18 hours later, the medium was irradiated with 32-48 mJ/cm² of UVA. Two hours before the irradiation, 100-200 µM of 6-O-dihomo-γ-linolenoyl ascorbate had been added to the medium but it was removed just before the irradiation and the medium was rinsed. UVA irradiation was performed in PBS but in the absence of the drug; after the irradiation, culture was continued in the 10% FBS containing DMEM and 24 hours after the irradiation, the cell viability was evaluated by a mitochondrial dehydrogenase activity assay method using 2-(4-iodophenyl)-3-(4-nitrophenyl)-5-(2,4-disulfophenyl)-2H-tetrazolium monosodium salt (WST-1). The results are shown in Fig. 2.

Obviously, the treatment with 200 µM of 6-O-dihomo-γ-linolenoyl ascorbate improved the viability of human skin keratocytes.

### Example 3:

### The effect of 6-O-dihomo-γ-linolenoyl ascorbate on the permeability of ascorbic acid to the tissue of human skin strips

A human skin section extracted from the right abdominal part of a 56-year-old male volunteer with an informed consent was vertically split into small strips, which were set in a modified Bronov diffusion cell chamber. With the underside of the skin immersed in DMEM (2 mL) to supply nutrients, the keratinized layer was aerated with 5% CO₂ to maintain the pH of the medium at 7.25. The keratinized layer was overlaid with double gauze of about 5 mm square soaked with 1 mL of the 6-O-dihomo-γ-linolenoyl ascorbate prepared in Example 2, provided that it had a concentration of 100 mM (3.38% w/w; PBS(-) solution).

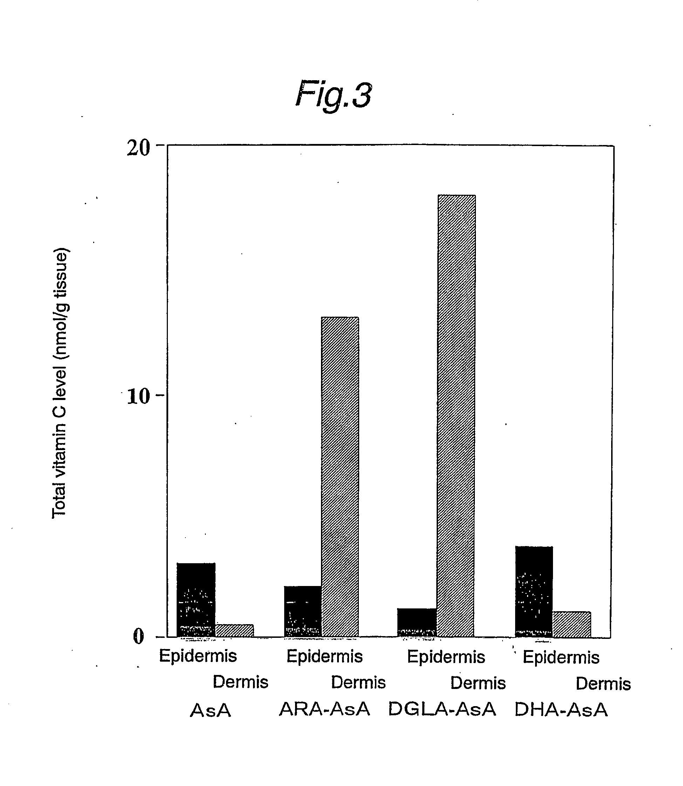

Four and 17 hours after the administration, an additional amount of 6-O-dihomo-γ-linolenoyl ascorbate was similarly administered at a concentration of 100 mM (3.38% w/w; PBS(-) solution); 24 hours later, the skin strips were excised out of the Bronov diffusion cell chamber, added with 10 volumes of a PBS(-) solution of 0.1% trypsin, and treated at 37 °C for 3 hours, followed by gentle stirring to separate the skin to the epidermis and the dermis. Under a bubble-free condition, treatment with a potter-type Teflon homogenizer and freeze-thaw cycles in liquid nitrogen were applied to each of the epidermis and the dermis to disrupt their cells. The homogenate was centrifuged and the supernatant was subjected to ultrafiltration. The total quantity of vitamin C (the sum of reduced and oxidized forms of vitamin C) and the quantity of reduced form of vitamin C in the epidermis and the dermis were measured; the total quantity of vitamin C was measured in the presence of 16 mM dithiothreitol as a reducing agent, whereas the quantity of reduced form of vitamin C was measured without adding any such reducing agent. The measured values were used to calculate the proportion of total vitamin C as occupied by reduced form of vitamin C. The results are shown in Fig. 3.

Similarly, 6-O-arachidonoyl ascorbate and 6-0-docosahexaenoyl ascorbate, as well as ascorbic acid (comparison) were evaluated and the results are also shown in Fig. 3.

As Fig. 3 shows, the amount of vitamin C in the dermis was increased markedly by 6-O-dihomo-γ-linolenoyl ascorbate and 6-O-arachidonoyl ascorbate, as compared with unmodified vitamin C.

### Example 4:

### Formulation of cream

Squalane (5.0 wt%), petrolatum (2.0 wt%), beeswax (0.5 wt%), sorbitan sesquioleate (0.8 wt%), polyoxyethylene oleyl ether (20E.O) (1.2 wt%), 1,3-butylene glycol (5.0 wt%), an antiseptic (q.s.), a flavoring agent (q.s.), and 6-O-dihomo-γ-linolenoyl ascorbate (1.0 wt%) were mixed and heated at 70 °C. In a separate step, ethyl alcohol (5.0 wt%) and purified water (59.5 wt%) were mixed and heated at 70 °C. The two mixtures were combined, cooled and added with a carboxyvinyl polymer (1% sol.) (20.0 wt%) to make a cream.

The same procedure was repeated to prepare a cream except that the 6-O-dihomo-γ-linolenoyl ascorbate was replaced by 6-O-arachidonoyl ascorbate.

The same procedure was repeated to prepare a cream except that the 6-O-dihomo-γ-linolenoyl ascorbate was replaced by 6-0-docosahexaenoyl ascorbate.

### Example 5:

### Formulation of toner

Polyoxyethylene (20E.O) sorbitan monolaurate (1.2 wt%), ethyl alcohol (8.0 wt%), 6-O-dihomo-γ-linolenoyl ascorbate (1.0 wt%), an antiseptic (q.s.), and a flavoring agent (q.s.) were mixed to form a solution. In a separate step, glycerin (5.0 wt%), 1,3-butylene glycol (6.5 wt%), and purified water (78.3 wt%) were mixed to form a solution. The two solutions were mixed to uniformity to prepare a toner.

The same procedure was repeated to prepare a toner except that the 6-O-dihomo-γ-linolenoyl ascorbate was replaced by 6-O-arachidonoyl ascorbate.

The same procedure was repeated to prepare a toner except that the 6-O-dihomo-y-linolenoyl ascorbate was replaced by 6-0-docosahexaenoyl ascorbate.

### Example 6:

### Formulation of emulsion

Polyoxyethylene (10E.O) sorbitan monostearate (1.0 wt%), polyoxyethylene (10E.O) sorbitan tetraoleate (1.0 wt%), glyceryl monostearate (1.0 wt%), stearic acid (0.5 wt%), behenyl alcohol (0.5 wt%), squalane (8.0 wt%), and 6-O-dihomo-γ-linolenoyl ascorbate (1.0 wt%) were mixed under heating at 70 °C. In a separate step, a carboxyvinyl polymer (0.1 wt%), ethyl alcohol (5.0 wt%), an antiseptic (q.s.), a flavoring agent (q.s.), and purified water (82.4 wt%) were mixed under heating at 70 °C. The two mixtures were combined to uniformity to prepare an emulsion.

The same procedure was repeated to prepare an emulsion except that the 6-O-dihomo-γ-linolenoyl ascorbate was replaced by 6-O-arachidonoyl ascorbate.

The same procedure was repeated to prepare an emulsion except that the 6-O-dihomo-γ-linolenoyl ascorbate was replaced by 6-0-docosahexaenoyl ascorbate.

### Example 7:

### Formulation of ointment

Triethanolamine (2.0 wt%), glycerin (5.0 wt), and purified water (70 wt%) were mixed under heating at 75 °C. In a separate step, stearic acid (18.0 wt), cetanol (4.0 wt%), and 6-O-dihomo-γ-linolenoyl ascorbate were mixed under heating at 75 °C. The former mixture was slowly added to the latter and the resulting mixture was cooled to prepare an ointment.

The same procedure was repeated to prepare an ointment except that the 6-O-dihomo-γ-linolenoyl ascorbate was replaced by 6-O-arachidonoyl ascorbate.

The same procedure was repeated to prepare an ointment except that the 6-O-dihomo-γ-linolenoyl ascorbate was replaced by 6-0-docosahexaenoyl ascorbate.

## Claims

1. A 6-O-PUFA ascorbate for use in a method for protecting skin from being damaged during exposure to UV radiation, wherein the permeability of ascorbic acid into cells in the dermis of the skin is improved, wherein the 6-O-PUFA ascorbate is 6-O-dihomo-γ-linolenoyl ascorbate or 6-O-arachidonoyl ascorbate.

2. A skin care product containing a 6-0-PUFA ascorbate for use in a method for protecting skin from being damaged during exposure to UV radiation, wherein the permeability of ascorbic acid into cells in the dermis of the skin is improved, wherein the 6-O-PUFA ascorbate is 6-O-dihomo-γ-linolenoyl ascorbate or 6-O-arachidonoyl ascorbate and wherein the 6-O-PUFA ascorbate is contained in an amount of 0.001-10 wt% in a base for cosmetics for external application.

3. The skin care product according to claim 2, which is selected from the group consisting of lotions, creams, toners, facial packs, cleansers, makeup cosmetics, scalp and hair care products, ointments, dispersions, and solutions for external application.

4. The skin care product according to claim 2 or 3, wherein the skin care product does not comprise a transdermal absorption enhancer.

5. Use of a 6-O-PUFA ascorbate for the manufacture of a skin care product for protecting skin from being damaged during exposure to UV radiation, wherein the permeability of ascorbic acid into cells in the dermis of the skin is improved, wherein the 6-O-PUFA ascorbate is 6-O-dihomo-γ-linolenoyl ascorbate or 6-O-arachidonoyl ascorbate.

6. The use according to claim 5, wherein the skin care product does not comprise a transdermal absorption enhancer.

## Patentansprüche

1. Ein 6-O-PUFA-Ascorbat zur Verwendung in einem Verfahren zum Schutz der Haut vor Schädigung während UV-Strahlenexposition, wobei die Permeabilität von Ascorbinsäure in die Zellen in der Dermis der Haut verbessert wird, wobei das 6-O-PUFA-Ascorbat 6-O-Dihomo-γ-linolenoylascorbat oder 6-O-Arachidonoylascorbat ist.

2. Ein Hautpflegeprodukt, enthaltend ein 6-O-PUFA-Ascorbat zur Verwendung in einem Verfahren zum Schutz der Haut vor Schädigung während UV-Strahlenexposition, wobei die Permeabilität von Ascorbinsäure in die Zellen in der Dermis der Haut verbessert wird, wobei das 6-O-PUFA-Ascorbat 6-O-Dihomo-γ-linolenoylascorbat oder 6-O-Arachidonoylascorbat ist und wobei das 6-O-PUFA-Ascorbat in einer Menge von 0,001-10 Gew.-% in einer Grundlage für Kosmetika zur äußeren Anwendung enthalten ist.

3. Das Hautpflegeprodukt nach Anspruch 2, welches ausgewählt ist aus der Gruppe bestehend aus Lotionen, Cremes, Gesichtswässer, Gesichtspackungen, Reinigungsmitteln, Make-Up-Kosmetika, Kopfhaut- und Haarpflegeprodukten, Salben, Dispersionen und Lösungen zur äußeren Anwendung.

4. Das Hautpflegeprodukt nach Anspruch 2 oder 3, wobei das Hautpflegeprodukt keinen transdermalen Absorptionsverstärker umfasst.

5. Verwendung eines 6-O-PUFA-Ascorbats zur Herstellung eines Hautpflegeprodukts zum Schutz der Haut vor Schädigung während UV-Strahlenexposition, wobei die Permeabilität von Ascorbinsäure in die Zellen in der Dermis der Haut verbessert wird, wobei das 6-O-PUFA-Ascorbat 6-O-Dihomo-γ-linolenoylascorbat oder 6-O-Arachidonoylascorbat ist.

6. Die Verwendung nach Anspruch 5, wobei das Hautpflegeprodukt keinen transdermalen Absorptionsverstärker umfasst.

## Revendications

1. Un 6-O-PUFA ascorbate pour utilisation dans une méthode de protection de la peau contre les lésions provoquées par les rayons UV, dans lequel la perméabilité de l'acide ascorbique dans les cellules du derme de la peau est améliorée et dans lequel le 6-O-PUFA ascorbate est le 6-O-dihomo-γ-linolénoyl ascorbate ou le 6-O-arachidonoyl ascorbate.

2. Un produit pour les soins de la peau contenant un 6-O-PUFA ascorbate pour utilisation dans une méthode de protection de la peau contre les lésions provoquées par les rayons UV, dans lequel la perméabilité de l'acide ascorbique dans les cellules du derme de la peau est améliorée, dans lequel le 6-0-PUFA ascorbate est le 6-O-dihomo-γ-linolénoyl ascorbate ou le 6-O-arachidonoyl ascorbate et dans lequel le 6-O-PUFA ascorbate est contenu dans une proportion comprise entre 0,001 et 10 % en poids dans une base pour cosmétiques destinés à l'application externe.

3. Le produit pour les soins de la peau selon la revendication 2, qui est choisi parmi le groupe se composant des lotions, crèmes, toniques, compresses pour le visage, nettoyants, cosmétiques pour le maquillage, produits de soins pour les cheveux et le cuir chevelu, pommades, dispersions et solutions pour l'application externe.

4. Le produit pour les soins de la peau selon la revendication 2 ou 3, dans lequel le produit pour les soins de la peau ne comprend pas de stimulateur d'absorption transdermique.

5. Utilisation d'un 6-O-PUFA pour la fabrication d'un produit pour les soins de la peau, destiné à protéger cette dernière contre les lésions provoquées par les rayons UV, dans lequel la perméabilité de l'acide ascorbique dans les cellules du derme de la peau est améliorée et dans lequel le 6-O-PUFA ascorbate est le 6-O-dihomo-γ-linolénoyl ascorbate ou le 6-O-arachidonoyl ascorbate.

6. L'utilisation selon la revendication 5, dans laquelle le produit pour les soins de la peau ne comprend pas de stimulateur d'absorption transdermique.
